(19) Europäisches Patentamt
European Patent Office
Office européen des brevets

(11) EP 3 798 185 A1

(12) # EUROPEAN PATENT APPLICATION
published in accordance with Art. 153(4) EPC

(43) Date of publication:
**31.03.2021 Bulletin 2021/13**

(21) Application number: **19807744.8**

(22) Date of filing: **22.05.2019**

(51) Int Cl.:
***C01B 25/36*** *(2006.01)* ***B01J 20/02*** *(2006.01)*
***B01J 20/28*** *(2006.01)* ***B01J 20/30*** *(2006.01)*

(86) International application number:
**PCT/JP2019/020340**

(87) International publication number:
**WO 2019/225667 (28.11.2019 Gazette 2019/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.05.2018 JP 2018098585**

(71) Applicant: **THE RESEARCH FOUNDATION FOR MICROBIAL DISEASES OF OSAKA UNIVERSITY**
**Suita-shi, Osaka 565-0871 (JP)**

(72) Inventors:
- **KANZAWA, Noriyuki**
  **Kanonji-shi, Kagawa 768-0065 (JP)**
- **KANO, Shunsuke**
  **Kanonji-shi, Kagawa 768-0065 (JP)**
- **MIYATAKE, Hiroshi**
  **Kanonji-shi, Kagawa 768-0065 (JP)**

(74) Representative: **Müller-Boré & Partner Patentanwälte PartG mbB**
**Friedenheimer Brücke 21**
**80639 München (DE)**

(54) **ALUMINUM PHOSPHATE COMPOUND**

(57) The present invention provides a substance which is useful as a carrier for protein purification. Since an aluminum phosphate compound that is obtained by a method for producing an aluminum phosphate compound, which comprises a step for obtaining a mixture that contains an aluminum phosphate compound by mixing an aqueous solution A containing a phosphate ion with an aqueous solution B containing an aluminum ion and a sulfate ion, and at least one of a potassium ion or a magnesium ion, exhibits excellent protein adsorption properties, this aluminum phosphate compound is useful as a carrier for protein purification.

FIG. 1

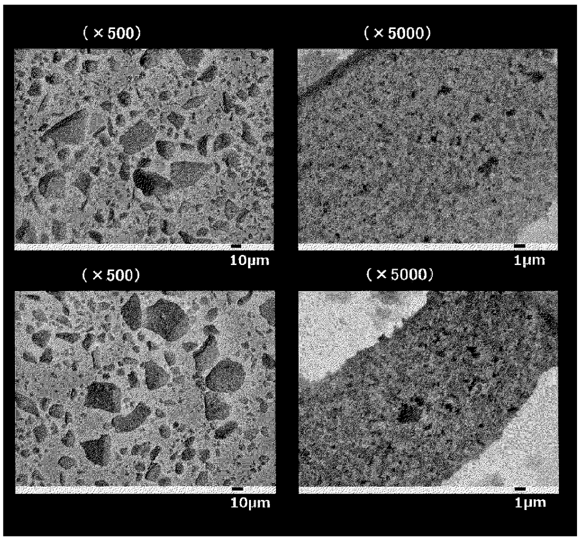


EP 3 798 185 A1

## Description

### TECHNICAL FIELD

The present invention relates to an aluminum phosphate compound. In particular, the present invention relates to an aluminum phosphate compound useful as a carrier for protein purification.

### BACKGROUND ART

Conventionally, purification methods such as salting-out method, centrifugation method and column chromatography method have been widely used for protein purification. In the case of vaccines among proteins, due to the need to reduce side reactions when inoculated into humans by removing medium components in a culture or extraction process of antigens, components derived from a host, and other components unnecessary for vaccines, and due to recent stricter formulation standards, particularly advanced purification methods are required. Specific examples of the method for purifying vaccine include polyethylene glycol salting-out method, ultracentrifugation method, sucrose density gradient centrifugation method, and chromatography method (for example, see Non-Patent Document 1).

### PRIOR ART DOCUMENT

### PATENT DOCUMENT

Non-Patent Document 1: General Incorporated Association, Japan Association of Vaccine Industries, 2017 Vaccine Basics - From production to distribution of vaccines

### SUMMARY OF THE INVENTION

### PROBLEMS TO BE SOLVED BY THE INVENTION

Although various protein purification methods have been developed, some vaccines and the like cannot be sufficiently purified, thus a new method useful for purification of proteins such as vaccines is required. The present inventors has investigated an inorganic compound such as aluminum phosphate as a purification carrier in order to search for a new protein purification method, but it did not fulfill a sufficient function as a carrier for protein purification.

Therefore, a main object of the present invention is to provide a substance useful as a carrier for protein purification.

### MEANS FOR SOLVING THE PROBLEM

As a result of intensive studies, the present inventors have found that, in the production of aluminum phosphate, an aluminum phosphate compound obtained by intentionally using an aqueous solution containing a specific ion which is not a constituent element of aluminum phosphate unexpectedly exhibits an excellent adsorption action for proteins, which is not found in aluminum phosphate used as a reagent, whereby the present invention has been completed. That is, the present invention provides the inventions of the following modes.

Item 1. A method for producing an aluminum phosphate compound, comprising a step for mixing an aqueous solution A that contains a phosphate ion and an aqueous solution B that contains an aluminum ion and a sulfate ion, and at least one of a potassium ion or a magnesium ion, to obtain a mixture containing an aluminum phosphate compound.

Item 2. The method for producing an aluminum phosphate compound according to Item 1, wherein the mixture has a pH of 3 to 4.

Item 3. The method for producing an aluminum phosphate compound according to Item 1 or 2, wherein the aqueous solution A is an aqueous monohydrogen phosphate solution.

Item 4. The method for producing an aluminum phosphate compound according to any of Items 1 to 3, wherein the aqueous solution B contains the potassium ion and the magnesium ion.

Item 5. The method for producing an aluminum phosphate compound according to any of Items 1 to 4, wherein the aqueous solution B is prepared by dissolving at least one of potassium aluminum sulfate or magnesium sulfate in water.

Item 6. The method for producing an aluminum phosphate compound according to any of Items 1 to 5, wherein the mixing is performed by adding the aqueous solution B to the aqueous solution A at a rate of 2% by volume/min or less.

Item 7. The method for producing an aluminum phosphate compound according to any of Items 1 to 6, wherein the aluminum phosphate compound is used for a carrier for protein purification.

Item 8. An aluminum phosphate compound obtained by the method for producing an aluminum phosphate compound according to any one of Items 1 to 7.

Item 9. An aluminum phosphate compound containing sulfur and at least one of 0.5% by weight or more of potassium or 0.01% by weight or more of magnesium, together with aluminum phosphate.

Item 10. The aluminum phosphate compound according to Item 9, wherein a content of sulfur is 0.5% by weight or more.

Item 11. The aluminum phosphate compound according to Items 9 or 10, wherein a content of aluminum is 10 to 16% by weight.

Item 12. The aluminum phosphate compound according to any of Items 9 to 11, wherein a water content is 15% by weight or more.

Item 13. The aluminum phosphate compound according to any of Items 9 to 12, wherein a content of calcium is 0.001% by weight or more.

Item 14. The aluminum phosphate compound according to any of Items 9 to 13, which is used as a carrier for protein purification.

Item 15. The aluminum phosphate compound according to any of Items 9 to 14, which is used for purification of a protein synthesized by a cell-free system, and/or a protein expressed in a host selected from the group consisting of bacteria, yeast, algae, an insect cell, a mammalian cell, and an animal cultured cell.

Item 16. The aluminum phosphate compound according to any one of Items 9 to 15, which is used for purification of a protein expressed in Escherichia coli as a host.

## ADVANTAGES OF THE INVENTION

According to the present invention, a substance useful as a carrier for protein purification is provided.

## BRIEF DESCRIPTION OF THE DRAWINGS

Fig. 1 shows a SEM photograph of an aluminum phosphate compound of the present invention obtained in Test Example 1.

Fig. 2 shows an adsorbed portion of an eluate when a protein derived from Escherichia coli (into which a gene was introduced to express a fluorescent protein added with a His tag) was adsorbed on the aluminum phosphate compound of the present invention (Example 3) and an adsorbed portion of an eluate when the protein was adsorbed on a commercially available aluminum phosphate (Comparative Example 1) in Test Example 3.

Fig. 3 shows results of electrophoresis of the eluates of Fig. 2 in Test Example 3.

Fig. 4 shows results of electrophoresis of the eluates of the adsorbed portions when a pneumococcal surface protein was adsorbed on the aluminum phosphate compound of the present invention (Examples 3 and 4) in Test Example 4.

## EMBODIMENTS OF THE INVENTION

### [1. Method for Producing Aluminum Phosphate Compound]

The method for producing an aluminum phosphate compound of the present invention includes mixing an aqueous solution A containing a phosphate ion, and an aqueous solution B containing an aluminum ion and a sulfate ion, and at least one of a potassium ion or a magnesium ion to obtain a mixed solution containing an aluminum phosphate compound. Hereinafter, the method for producing an aluminum phosphate compound of the present invention will be described in detail.

### [1-1. Aqueous Solution A]

The aqueous solution A contains phosphate ions. The aqueous solution A can be prepared as an aqueous solution in which a phosphoric acid-containing compound is dissolved. Examples of the phosphoric acid-containing compound include phosphoric acid, non-hydrogen phosphate, monohydrogen phosphate, dihydrogen phosphate, and the like.

The phosphoric acid is orthophosphoric acid. The non-hydrogen phosphate is a phosphate that is not a hydrogen salt, and examples thereof include ammonium phosphate ($(NH_4)_3PO_4$), trisodium phosphate ($Na_3PO_4$), tripotassium phosphate ($K_3PO_4$), ammonium magnesium phosphate ($MgNH_4PO_4$), hydroxyapatite ($Ca_{10}(PO_4)_6(OH)_2$), and the like. The monohydrogen phosphate is a monohydrogen phosphate, and examples thereof include diammonium hydrogen phosphate ($(NH_4)_2HPO_4$), disodium hydrogenphosphate ($Na_2HPO_4$), dipotassium hydrogenphosphate ($K_2HPO_4$), mag-

nesium monohydrogen phosphate ($MgHPO_4$), calcium monohydrogen phosphate ($CaHPO_4$), and the like. The dihydrogen phosphate is a dihydrogen phosphate, and examples thereof include ammonium dihydrogen phosphate ($NH_4H_2PO_4$), sodium dihydrogen phosphate ($NaH_2PO_4$), potassium dihydrogen phosphate ($KH_2PO_4$), and the like.

The phosphoric acid-containing compound may be an anhydrate or a hydrate. The hydration number of the hydrate is not particularly limited. These phosphoric acid-containing compounds may be used alone or in combination of two or more.

Among the above-mentioned phosphoric acid-containing compounds, in the present invention, examples thereof preferably include monohydrogen phosphate, and more preferably include disodium hydrogenphosphate ($Na_2HPO_4$), from the viewpoint of obtaining better protein adsorption properties of the aluminum phosphate compound and the like.

The concentration of phosphate ions in the aqueous solution A is not particularly limited, and the total amount of phosphate ions constituting the above-mentioned phosphate-containing compound used for the preparation of the aqueous solution A includes, for example, 0.01 mol/L or more, preferably 0.05 mol/L or more, more preferably 0.1 mol/L or more, and further preferably 0.2 mol/L or more, from the viewpoint of reacting with the aqueous solution B to efficiently obtain an aluminum phosphate compound and the like, and includes, for example, 0.6 mol/L or less, preferably 0.45 mol/L or less, and more preferably 0.3 mol/L or less, from the viewpoint of obtaining better protein adsorption properties of the aluminum phosphate compound and the like. Examples of specific range of the total amount of phosphate ions constituting the phosphoric acid-containing compound used for the preparation of the aqueous solution A include 0.01 to 0.6 mol/L, 0.01 to 0.45 mol/L, 0.01 to 0.3 mol/L, 0.05 to 0.6 mol/L, 0.05 to 0.45 mol/L, 0.05 to 0.3 mol/L, 0.1 to 0.6 mol/L, 0.1 to 0.45 mol/L, 0.1 to 0.3 mol/L, 0.2 to 0.6 mol/L, 0.2 to 0.45 mol/L, and 0.2 to 0.3 mol/L.

Although the liquid properties of the aqueous solution A are not particularly limited, it can be prepared, for example, as a basic solution. The aqueous solution A as a basic solution may be prepared by dissolving the above-mentioned phosphoric acid-containing compound in water, may be prepared by dissolving the above-mentioned phosphoric acid compound in water and adjusting pH using one or more metal hydroxides such as sodium hydroxide, potassium hydroxide, magnesium hydroxide and calcium hydroxide, or may be prepared by dissolving the above-mentioned phosphorus compound in a basic aqueous solution of the metal hydroxide prepared in advance. Specific pH of the aqueous solution A (23°C, the same applies below) can be preferably determined in consideration of pH of the aqueous solution B so that pH of the mixture obtained by mixing with the aqueous solution B described later is 3 to 4. Specifically, examples of the lower limit of the pH of the aqueous solution A include 7.0 or higher, and preferably 7.5 or higher, and examples of the upper limit thereof include 14.0 or lower, and preferably 9.5 or lower. Examples of specific range of the pH of the aqueous solution A include 7.0 to 14.0, 7.0 to 9.5, 7.5 to 14.0, and 7.5 to 9.5.

The method for dissolving the phosphoric acid-containing compound is not particularly limited, and examples thereof include stirring, shaking, ultrasonic dissolution, and the like. Moreover, the conditions of temperature and pressure when dissolving the phosphoric acid-containing compound are not also particularly limited, and examples thereof include conditions such as normal temperature and normal pressure and warm normal pressure.

The aqueous solution A can be prepared at room temperature, and the aqueous solution A is preferably obtained by filtering with the prepared solution by a filter. The pore size of the filter used includes, for example, 0.2 to 1.0 μm, preferably 0.2 to 0.5 μm, and more preferably 0.2 to 0.4 μm.

[1-2. Aqueous Solution B]

The aqueous solution B contains an aluminum ion and a sulfate ion, and at least one of a potassium ion or a magnesium ion. Thus, in the present invention, the aluminum phosphate compound obtained by intentionally using an aqueous solution containing a specific ion which is not a constituent element of aluminum phosphate can be obtained as a useful substance as a carrier for protein purification, which has an excellent adsorption action on proteins. From the viewpoint of obtaining better protein adsorption properties of the aluminum phosphate compound and the like, the aqueous solution B preferably contains an aluminum ion, a sulfate ion, a potassium ion, and a magnesium ion.

The aqueous solution B can be prepared as an aqueous solution in which one or two or more salts containing an aluminum ion and a sulfate ion, and at least one of a potassium ion or a magnesium ion are dissolved. The salt may be a single salt or a double salt.

As the salt for preparing the aqueous solution B, a plurality of types of salts each containing an aluminum ion and a sulfate ion, and at least one of a potassium ion or a magnesium ion may be combined, or salts containing a plurality of types of ions may be single or combined. When using two or more types of salts for preparing the aqueous solution B, it is preferable that both salts have a sulfate ion as an anion, from the viewpoint of obtaining better protein adsorption properties of the aluminum phosphate compound and the like.

Examples of the salts containing aluminum ions include aluminum hydroxide ($Al(OH)_3$), aluminum oxide ($Al_2O_3$), aluminum sulfate ($Al_2(SO_4)_3$), aluminum chloride ($AlCl_3$), aluminum nitrate ($Al(NO_3)_3$), potassium aluminum sulfate ($AlK(SO_4)_2$), magnesium aluminum sulfate ($AlMgSO_4$), and the like, and preferably include aluminum sulfate ($Al_2(SO_4)_3$), potassium aluminum sulfate ($AlK(SO_4)_2$), and magnesium aluminum sulfate ($AlMgSO_4$), more preferably include potas-

sium aluminum sulfate ($AlK(SO_4)_2$) and magnesium aluminum sulfate ($AlMgSO_4$), and particularly preferably include potassium aluminum sulfate ($AlK(SO_4)_2$).

Examples of the salts containing sulfate ions include, in addition to aluminum sulfate ($Al_2(SO_4)_3$), potassium aluminum sulfate ($AlK(SO_4)_2$) and magnesium aluminum sulfate ($AlMgSO_4$) described above, potassium sulfate, magnesium sulfate and the like, and more preferably include potassium aluminum sulfate ($AlK(SO_4)_2$) and magnesium sulfate.

Examples of the salts containing potassium ions include, in addition to potassium aluminum sulfate ($AlK(SO_4)_2$) and potassium sulfate described above, potassium hydroxide (KOH), potassium chloride (KCl), potassium nitrate ($KNO_3$) and the like, preferably include potassium aluminum sulfate ($AlK(SO_4)_2$) and potassium sulfate, and more preferably include potassium aluminum sulfate ($AlK(SO_4)_2$).

Examples of the salts containing magnesium ions include, in addition to magnesium aluminum sulfate ($AlMgSO_4$) and magnesium sulfate described above, magnesium hydroxide ($Mg(OH)_2$), magnesium chloride ($MgCl_2$) and magnesium nitrate ($Mg(NO_3)_2$), preferably include magnesium aluminum sulfate ($AlMgSO_4$) and magnesium sulfate, and more preferably include magnesium sulfate.

The above-mentioned salt may be an anhydrate or a hydrate. The hydration number of the hydrate is not particularly limited.

Among the above-mentioned salts, in the present invention, examples preferably include a combination of potassium aluminum sulfate ($AlK(SO_4)_2$) and magnesium sulfate ($MgSO_4$), from the viewpoint of obtaining better protein adsorption properties of the aluminum phosphate compound and the like.

The concentration of aluminum ions in the aqueous solution B is not particularly limited, and the total amount of aluminum ions constituting the above-mentioned salt used for the preparation of the aqueous solution B includes, for example, 0.01 mol/L or more and preferably 0.05 mol/L or more, from the viewpoint of reacting with the aqueous solution A to efficiently obtain an aluminum phosphate compound and the like, and includes, for example, 0.3 mol/L or less and preferably 0.2 mol/L or less, from the viewpoint of obtaining better protein adsorption properties of the aluminum phosphate compound and the like. Examples of specific range of the total amount of aluminum ions constituting the salt used for the preparation of the aqueous solution B include 0.01 to 0.3 mol/L, 0.01 to 0.2 mol/L, 0.05 to 0.3 mol/L, and 0.05 to 0.2 mol/L.

Further, as the amount of aluminum ions in the aqueous solution B, examples of the lower limit of the total amount of aluminum ions constituting the above-mentioned salt used for the preparation of the aqueous solution B include 0.1 mol or more and preferably 0.2 mol or more, per mol of phosphate ions constituting the phosphoric acid-containing compound used for the preparation of the aqueous solution A, and examples of the upper limit include 0.5 mol or less and preferably 0.3 mol or less, per mol of phosphate ions constituting the phosphoric acid-containing compound used for the preparation of the aqueous solution A, from the viewpoint of obtaining better protein adsorption properties of the aluminum phosphate compound and the like. Examples of specific range of the total amount of aluminum ions constituting the above-mentioned salt used for the preparation of the aqueous solution B include 0.1 to 0.5 mol, 0.1 to 0.3 mol, 0.2 to 0.5 mol, and 0.2 to 0.3 mol, per mol of phosphate ions constituting the phosphoric acid-containing compound used for the preparation of the solution A.

The concentration of sulfate ions in the aqueous solution B is not particularly limited, and the lower limit of the total amount of sulfate ions constituting the above-mentioned salt used for the preparation of the aqueous solution B includes, for example, 0.1 mol/L or more and preferably 0.15 mol/L or more, and the upper limit includes, for example, 1 mol/L or less, preferably 0.5 mol/L or less, and more preferably 0.3 mol/L or less, from the viewpoint of reacting with the aqueous solution A to efficiently obtain an aluminum phosphate compound, and/or obtaining better protein adsorption properties of the aluminum phosphate compound and the like. Examples of specific range of the total amount of sulfate ions constituting the above-mentioned salt used for the preparation of the aqueous solution B include 0.1 to 1 mol/L or less, 0.1 to 0.5 mol/L, 0.1 to 0.3 mol/L, 0.15 to 1 mol/L, 0.15 to 0.5 mol/L, and 0.15 to 0.3 mol/L.

Further, as the amount of sulfate ions in the aqueous solution B, examples of the lower limit of the total amount of sulfate ions constituting the above-mentioned salt used for the preparation of the aqueous solution B include 0.5 mol or more and preferably 0.7 mol or more, per mol of phosphate ions constituting the phosphoric acid-containing compound used for the preparation of the aqueous solution A, and examples of the upper limit include 2 mol or less and preferably 1 mol or less, per mol of phosphate ions constituting the phosphoric acid-containing compound used for the preparation of the aqueous solution A, from the viewpoint of obtaining better protein adsorption properties of the aluminum phosphate compound and the like. Examples of specific range of the total amount of sulfate ions constituting the above-mentioned salt used for the preparation of the aqueous solution B include 0.5 to 2 mol, 0.5 to 1 mol, 0.7 to 2 mol, and 0.7 to 1 mol, per mol of phosphate ions constituting the phosphoric acid-containing compound used for the preparation of the aqueous solution A.

When the aqueous solution B contains potassium ions, the concentration of potassium ions in the aqueous solution B is not particularly limited, and the lower limit of the total amount of potassium ions constituting the above-mentioned salt used for the preparation of the aqueous solution B includes, for example, 0.01 mol/L or more and preferably 0.05 mol/L or more, and the upper limit includes, 0.2 mol/L or less and preferably 0.1 mol/L, from the viewpoint of reacting with the aqueous solution A to efficiently obtain an aluminum phosphate compound, and/or obtaining better protein adsorption properties of the aluminum phosphate compound and the like. Examples of specific range of the total amount of potassium ions constituting the above-mentioned salt used in the preparation of the aqueous solution B include 0.01 to 0.2 mol/L, 0.01 to 0.1 mol/L, 0.05 to 0.2 mol/L, and 0.05 to 0.1 mol/L.

Further, when the aqueous solution B contains potassium ions, as the amount of potassium ions in the aqueous solution B, examples of the lower limit of the total amount of potassium ions constituting the above-mentioned salt used for the preparation of the aqueous solution B include 0.1 mol or more and preferably 0.2 mol or more, per mol of phosphate ions constituting the phosphoric acid-containing compound used for the preparation of the aqueous solution A, and examples of the upper limit include 2.0 mol or less, preferably 1.2 mol or less, more preferably 0.8 mol or less, and further preferably 0.4 mol or less, per mol of phosphate ions constituting the phosphoric acid-containing compound used for the preparation of the aqueous solution A, from the viewpoint of obtaining better protein adsorption properties of the aluminum phosphate compound and the like. Examples of specific range of the total amount of potassium ions constituting the above-mentioned salt used for the preparation of the aqueous solution B include 0.1 to 2.0 mol, 0.1 to 1.2 mol, 0.1 to 0.8 mol, 0.1 to 0.4 mol, 0.2 to 2.0 mol, 0.2 to 1.2 mol, 0.2 to 0.8 mol, and 0.2 to 0.4 mol, per mol of phosphate ions constituting the phosphoric acid-containing compound used for the preparation of the aqueous solution A.

When the aqueous solution B contains magnesium ions, the concentration of magnesium ions in the aqueous solution B is not particularly limited, and the lower limit of the total amount of magnesium ions constituting the above-mentioned salt used for the preparation of the aqueous solution B includes, for example, 0.05 mol/L or more and preferably 0.07 mol/L or more, and the upper limit includes, for example, 0.2 mol/L or less and preferably 0.1 mol/L or less, from the viewpoint of reacting with the aqueous solution A to efficiently obtain an aluminum phosphate compound, and/or obtaining better protein adsorption properties of the aluminum phosphate compound and the like. Examples of specific range of the total amount of magnesium ions constituting the above-mentioned salt used in the preparation of the aqueous solution B include 0.05 to 0.2 mol/L, 0.05 to 0.1 mol/L, 0.07 to 0.2 mol/L, and 0.07 to 0.1 mol/L.

Further, when the aqueous solution B contains magnesium ions, as the amount of magnesium ions in the aqueous solution B, examples of the lower limit of the total amount of magnesium ions constituting the above-mentioned salt used for the preparation of the aqueous solution B include 0.1 mol or more and preferably 0.2 mol or more, per mol of phosphate ions constituting the phosphoric acid-containing compound used for the preparation of the aqueous solution A, and examples of the upper limit include 2.0 mol or less, preferably 1.2 mol or less, and more preferably 0.5 mol or less, per mol of phosphate ions constituting the phosphoric acid-containing compound used for the preparation of the aqueous solution A, from the viewpoint of obtaining better protein adsorption properties of the aluminum phosphate compound and the like. Examples of specific range of the total amount of magnesium ions constituting the above-mentioned salt used in the preparation of the aqueous solution B include 0.1 to 2.0 mol, 0.1 to 1.2 mol, 0.1 to 0.5 mol, 0.2 to 2.0 mol, 0.2 to 1.2 mol, and 0.2 to 0.5 mol.

Although the liquid properties of the aqueous solution B are not particularly limited, it can be prepared, for example, as an acidic solution. The aqueous solution B as an acidic solution may be prepared by dissolving the above-mentioned salt in water, may be prepared by dissolving the above-mentioned salt in water and adjusting pH using one or more mineral acids such as hydrochloric acid, nitric acid, sulfuric acid and phosphoric acid, or may be prepared by dissolving the above-mentioned salt in an acidic aqueous solution of the mineral acid prepared in advance. Specific pH of the aqueous solution B can be preferably determined in consideration of pH of the aqueous solution A so that pH of the mixture obtained by mixing with the aqueous solution A described above is 3 to 4. Specifically, examples of the lower limit of the pH of the aqueous solution B include 1.0 or higher and preferably 2.5 or higher, and examples of the upper limit thereof include 7.0 or lower and preferably 3.5 or lower. Examples of specific range of the pH of the aqueous solution B include 1.0 to 7.0, 1.0 to 3.5, 2.5 to 7.0, and 2.5 to 3.5.

The method of dissolving the above-mentioned salt is not particularly limited, and examples thereof include stirring, shaking, ultrasonic dissolution, and the like. Moreover, the conditions of temperature and pressure when dissolving the above-mentioned salt are not also particularly limited, and examples thereof include conditions such as normal temperature and normal pressure and warm normal pressure.

The aqueous solution B can be prepared at room temperature, and the aqueous solution B is preferably obtained by filtering with the prepared solution by a filter. The pore size of the filter used includes, for example, 0.2 to 1.0 μm, preferably 0.2 to 0.5 μm, and more preferably 0.2 to 0.4 μm.

[1-3. Mixing]

The aqueous solution A and the aqueous solution B produce an aluminum phosphate compound by mixing. From the viewpoint of obtaining better protein adsorption properties of the aluminum phosphate compound and the like, they can be preferably mixed by adding the aqueous solution B to the aqueous solution A. The addition method is not particularly limited, and examples thereof include batch addition, dropwise addition, divided addition, and any combinations thereof, but from the viewpoint of obtaining better protein adsorption properties of the aluminum phosphate compound and the like, examples preferably include dropwise addition, divided addition, and combinations thereof, and more preferably include dropwise addition. From the same viewpoint, it is further preferable that the addition rate of the aqueous solution B to the aqueous solution A is 2% by volume/min or less. The addition rate of 2% by volume/min refers to a rate at which the amount of the aqueous solution B added per minute is 2% by volume of a volume of the aqueous solution A before addition. Further, the addition rate of the aqueous solution B to the aqueous solution A can be set to 0.1% by volume/min or more, from the viewpoint of reacting the aqueous solution A and the aqueous solution B to efficiently obtain an aluminum phosphate compound and the like. From the viewpoint of more preferably obtaining these effects, examples of the lower limit of the dropping rate of the aqueous solution B to the aqueous solution A include 1% by volume/min or more and preferably 1.6% by volume/min or more, and examples of the preferable upper limit include 1.8% by volume/min. Examples of specific range of the dropping rate of the aqueous solution B to the aqueous solution A include 1 to 2% by volume/min, 1 to 1.8% by volume/min, 1.6 to 2% by volume/min, and 1.6 to 1.8% by volume/min.

The pH of the obtained mixture of the aqueous solution A and the aqueous solution B is not particularly limited, but examples thereof preferably include 3 to 4, from the viewpoint of obtaining better protein adsorption properties of the aluminum phosphate compound and the like. By mixing the aqueous solution A and the aqueous solution B, the aluminum phosphate compound can be obtained as a precipitate or a gel.

[1-4. Post-processing]

The produced aluminum phosphate compound is subjected to post-processing such as recovery from the mixture, washing and drying. The methods of recovery from the mixture, washing and drying are not particularly limited. Examples of the method of recovery from the mixture include filtration and centrifugation, and preferably centrifugation. The recovered aluminum phosphate compound can be obtained in the form of a precipitate or a gel. Examples of the washing method include suspension in an aqueous solution and centrifugation. Preferably, examples thereof include a method in which the recovered aluminum phosphate compound is suspended in physiological saline, stirred, and then centrifuged. Such a washing operation can be performed once or multiple times. The washed aluminum phosphate compound can be obtained in the form of a precipitate or gel. The drying conditions are not particularly limited, and examples thereof include 55 to 65°C for 1 to 2 hours, while it also depends on the production scale.

[2. Aluminum Phosphate Compound]

The aluminum phosphate compound of the present invention contains sulfur and at least one of 0.5% by weight or more of potassium or 0.01% by weight or more of magnesium, together with aluminum phosphate. As described above, the aluminum phosphate compound of the present invention is constituted by containing impurities other than aluminum phosphate, thereby exhibiting excellent protein adsorption properties, and becomes a useful substance as a carrier for protein purification. Hereinafter, the aluminum phosphate compound of the present invention will be described in detail.

[2-1. Composition]

The aluminum phosphate compound of the present invention contains not only aluminum phosphate but also sulfur and at least one of 0.5% by weight or more of potassium or 0.01% by weight or more of magnesium. From the viewpoint of obtaining better protein adsorption properties and the like, the aluminum phosphate compound of the present invention preferably contains aluminum phosphate, sulfur, 0.5% by weight or more of potassium, and 0.01% by weight or more of magnesium.

The content of sulfur (sulfur atom) in the aluminum phosphate compound is not particularly limited, but examples of the lower limit include 0.5% by weight or more, preferably 1.0% by weight or more, and more preferably 1.3% by weight or more, and examples of the upper limit include 5.0% by weight or less, preferably 3.0% by weight or less, and more preferably 2.0% by weight or less, from the viewpoint of obtaining better protein adsorption properties and the like. Examples of specific range of the content of sulfur (sulfur atom) in the aluminum phosphate compound include 0.5 to 5.0% by weight, 0.5 to 3.0% by weight, 0.5 to 2.0% by weight, 1.0 to 5.0% by weight, 1.0 to 3.0% by weight, 1.0 to 2.0% by weight, 1.3 to 5.0% by weight, 1.3 to 3.0% by weight, and 1.3 to 2.0% by weight.

As the content of potassium (potassium atom) in the aluminum phosphate compound, examples of the lower limit include 0.5% by weight or more, preferably 1.0% by weight or more, and more preferably 1.3% by weight or more, and examples of the upper limit include 5.0% by weight or less, preferably 3.0% by weight or less, and more preferably 2.0% by weight or less, from the viewpoint of obtaining better protein adsorption properties and the like. Examples of specific range of the content of potassium (potassium atom) in the aluminum phosphate compound include 0.5 to 5.0% by weight, 0.5 to 3.0% by weight, 0.5 to 2.0% by weight, 1.0 to 5.0% by weight, 1.0 to 3.0% by weight, 1.0 to 2.0% by weight, 1.3 to 5.0% by weight, 1.3 to 3.0% by weight, and 1.3 to 2.0% by weight.

As the content of magnesium (magnesium atom) in the aluminum phosphate compound, examples of the lower limit include 0.01% by weight or more, preferably 0.10% by weight or more, and more preferably 0.14% by weight or more, and examples of the upper limit include 1.0% by weight or less, preferably 0.5% by weight or less, and more preferably 0.2% by weight or less, from the viewpoint of obtaining better protein adsorption properties and the like. Examples of specific range of the content of magnesium (magnesium atom) in the aluminum phosphate compound include 0.01 to 1.0% by weight, 0.01 to 0.5% by weight, 0.01 to 0.2% by weight, 0.10 to 1.0% by weight, 0.10 to 0.5% by weight, 0.10 to 0.2% by weight, 0.14 to 1.0% by weight, 0.14 to 0.5% by weight, and 0.14 to 0.2% by weight.

The aluminum phosphate compound may contain other impurities in addition to the above impurities. The other impurities include water and calcium, and the like.

As the water content in the aluminum phosphate compound, examples of the lower limit include preferably 15% by weight or more and more preferably 17% by weight or more, and examples of the upper limit include 40% by weight or less, preferably 30% by weight or less, and more preferably 20% by weight or less, from the viewpoint of obtaining better protein adsorption properties and the like. Examples of specific range of the water content in the aluminum phosphate compound include 15 to 40% by weight, 15 to 30% by weight, 15 to 20% by weight, 17 to 40% by weight, 17 to 30% by weight, and 17 to 20% by weight. The water content in the aluminum phosphate compound can be measured using a thermogravimetric-mass spectroscope (TG-MS) as in Examples described later.

As the content of calcium (calcium atom) in the aluminum phosphate compound, examples of the lower limit include 0.001% by weight or more and preferably 0.003% by weight or more, and examples of the upper limit include 0.1% by weight or less, preferably 0.01% by weight or less, and more preferably 0.006% by weight or less, from the viewpoint of obtaining better protein adsorption properties and the like. Examples of specific range of the content of calcium (calcium atom) in the aluminum phosphate compound include 0.001 to 0.1% by weight, 0.001 to 0.01% by weight, 0.001 to 0.006% by weight, 0.003 to 0.1% by weight, 0.003 to 0.01% by weight, and 0.003 to 0.006% by weight.

The content of aluminum in the aluminum phosphate compound is not particularly limited as long as it contains the above-mentioned impurities, but examples of the lower limit include preferably 10% by weight or more and more preferably 12% by weight or more, and examples of the upper limit include 35% by weight or less, more preferably 30% by weight or less, further preferably 28% by weight or less, still more preferably 16% by weight or less, and particularly preferably 15% by weight or less, from the viewpoint of obtaining better protein adsorption properties and the like. Examples of specific range of the content of aluminum in the aluminum phosphate compound include 10 to 35% by weight, 10 to 30% by weight, 10 to 28% by weight, 10 to 16% by weight, 10 to 15% by weight, 12 to 35% by weight, 12 to 30% by weight, 12 to 28% by weight, 12 to 16% by weight, 12 to 15% by weight, 10 to 35% by weight, 10 to 30% by weight, 10 to 28% by weight, 10 to 16% by weight, and 10 to 15% by weight.

As the molar ratio of aluminum to phosphorus (A1 (mol)/P (mol)) in the aluminum phosphate compound, examples of the lower limit include 0.1 or more, preferably 0.5 or more, more preferably 0.7 or more, and further preferably 0.9 or more, and examples of the upper limit include 2.0 or less, preferably 1.5 or less, and more preferably 1.0 or less. Examples of specific range of the molar ratio of aluminum to phosphorus in the aluminum phosphate compound include 0.1 to 2.0, 0.1 to 1.5, 0.1 to 1.0, 0.5 to 2.0, 0.5 to 1.5, 0.5 to 1.0, 0.7 to 2.0, 0.7 to 1.5, 0.7 to 1.0, 0.9 to 2.0, 0.9 to 1.5, and 0.9 to 1.0.

[2-2. Physical Characteristics]

The aluminum phosphate compound of the present invention is amorphous and has fine irregularities on the surface. The fine irregularities can be confirmed, for example, at 5,000 times by electron microscope (SEM) observation. Specifically, the aluminum phosphate compound has a cumulative pore volume of preferably 1.0 to 1.5 mL/g and more preferably 1.0 to 1.2 mL/g; an average pore diameter of preferably 0.01 to 0.1 μm and more preferably 0.01 to 0.04 μm; a bulk density of preferably 0.5 to 1.0 g/mL and more preferably 0.63 to 1.0 g/mL; an apparent density of preferably 2.0 to 2.5 g/mL and more preferably 2.1 to 2.4 g/mL; and a porosity of preferably 60 to 80% and more preferably 60 to 70%, from the viewpoint of obtaining better protein adsorption properties and the like. The cumulative pore volume, average pore diameter, bulk density, apparent density, and porosity can be measured by mercury porosimetry as described in Examples described later. The mercury porosimetry is a known method as a method that can determine pore distribution from a pressure and an amount of injection when mercury is injected into the pores, and also can determine bulk density, apparent density and porosity, from a volume of cell, a volume of mercury obtained from a weight of mercury in the cell, and a pore volume.

[2-3. Production]

The method for producing an aluminum phosphate compound of the present invention is not particularly limited as long as it is a method capable of obtaining an aluminum phosphate compound with the above composition, but it is preferably produced by "1. Method for Producing Aluminum Phosphate Compound" described above. Incidentally, in order to incorporate calcium in the above-mentioned content, a salt containing calcium may be used as a raw material in the method for producing an aluminum phosphate compound, but preferably, calcium can be contained as a component derived from water retained at the water content described above, without using the salt containing calcium as the raw material.

[2-4. Application]

The aluminum phosphate compound of the present invention is useful as a carrier for protein purification because it has excellent protein adsorption properties. It can be suitably used as a carrier for purification of proteins, particularly vaccines, which could not be sufficiently purified by conventional carriers for purification. The type of vaccine is not particularly limited, and examples thereof include influenza HA vaccine, polio vaccine, Japanese encephalitis vaccine, pertussis vaccine, mumps vaccine, tetanus vaccine, diphtheria vaccine, hepatitis A vaccine, hepatitis B vaccine, pneumococcal vaccine, influenza type b vaccine, human papillomavirus vaccine, rotavirus vaccine, and the like.

The aluminum phosphate compound of the present invention can be used as a carrier for purifying a protein synthesized by a cell-free system, and/or a protein expressed using bacteria, yeast, algae, an insect cell, a mammalian cell, an animal cultured cell or the like as a host.

Further, the aluminum phosphate compound of the present invention also can effectively remove endotoxin derived from Escherichia coli, which is a serious problem for quality control of pharmaceuticals. Therefore, the aluminum phosphate compound of the present invention is particularly suitable for purification of a protein expressed in Escherichia coli as a host.

Furthermore, since the aluminum phosphate compound of the present invention has an excellent affinity with a histidine tag, it is also suitable for purification of a protein synthesized or expressed with a histidine tag attached thereto. The aluminum phosphate compound of the present invention exhibits excellent specificity even for proteins to which a histidine tag has not been added, and thus is suitable for purification of a wide range of proteins regardless of whether or not a histidine tag is added.

EXAMPLES

Hereinafter, the present invention will be described in detail with reference to Examples and Comparative Examples, but the present invention is not limited thereto.

[Test Example 1: Production of Aluminum Phosphate Compound]

Aqueous solution A and aqueous solution B having the following compositions were prepared at normal temperature (room temperature 23°C) and normal pressure, and filtered with a filter with a pore size of 0.22 μm, respectively.

- Aqueous solution A

35.9 g of disodium hydrogen phosphate dodecahydrate was dissolved by adding water and stirring to prepare a total of 1000 mL of aqueous solution (phosphate ion concentration: 0.25 mol/L, pH (23°C): 9.0 to 9.5).

- Aqueous solution B

28.4 g of potassium aluminum sulfate dodecahydrate and 9.86 g of magnesium sulfate heptahydrate were dissolved by adding water and stirring to prepare a total of 1000 mL of aqueous solution (aluminum ion concentration: 0.06 mol/L, sulfate ion concentration: 0.2 mol/L, potassium ion concentration: 0.06 mol/L, magnesium ion concentration: 0.08 mol/L, pH (23°C): 3.0 to 3.5).

The aqueous solution A and the aqueous solution B were mixed at a volume ratio of 1 : 1. Specifically, while stirring the aqueous solution A at the stirring rate shown in Table 1, a mixture obtained by adding the aqueous solution B dropwise at the addition rate shown in Table 1 (pH (23°C): 3.0 to 3.5) was centrifuged at 5,000 × g for 15 minutes, and a gel-like precipitate was collected. The collected gel-like substance was put into 0.9 w/v% physiological saline, suspended and washed using a homogenizer, and then centrifuged at 5,000 × g for 15 minutes, and a gel-like substance was collected. Suspension of the gel-like substance in physiological saline and centrifugation were repeated once more under the same conditions. The obtained gel-like substance was spread on a tray and dried at 60°C for 1 to 2 hours. This gave a dry gel, which was white crystals. The dried gel was manually crushed and pulverized to obtain an aluminum phosphate compound.

[Table 1]

| | Stirring rate of aqueous solution A | Addition rate of aqueous solution B | Time required for mixing |
|---|---|---|---|
| Example 1 | 700 rpm | 1.7% by volume/min | 1 hr |
| Example 2 | 200 rpm | (Batch addition) | about 2 sec |

[Test Example 2: Analysis of Aluminum Phosphate Compound]

The aluminum phosphate compounds of Examples 1 and 2 were analyzed as follows.

(1) Elemental analysis (Al, K, P, S, Ca, Mg, Na) measurement

- Measurement method: Acid decomposition/ICP-AES method
- Measuring device: Agilent 5110 (manufactured by Agilent Technologies Japan, Ltd.)
- Results: The results of elemental analysis are shown in Tables 2 and 3 below. As shown in the table below, Al and P were detected as main components, and in addition, Na and Cl (not shown in the table) were detected. Na and Cl were also detected and confirmed by XRD described later, thus it can be inferred that they are derived from NaCl. The compound containing Al and P as the main components were determined to be amorphous by XRD described later. The amorphous composition of the detected element includes anhydrous aluminum phosphate, aluminum phosphate hydrate, and aluminum hydrogen phosphate. It is considered not to be anhydrous aluminum phosphate since it contains water determined by thermogravimetric-mass spectrometry described later. Further, when aluminum hydrogen phosphate should have deliquescent properties, deliquescent behavior was not observed at each measurement, so it is considered not to be aluminum hydrogen phosphate. Therefore, the obtained amorphous substance is considered to be aluminum phosphate hydrate. From the water content and the quantitative value of Al, the hydration number is considered to be 1.5 to 2.

[Table 2]

| Unit: % by weight | | | | | | |
|---|---|---|---|---|---|---|
| | Example 1 | | | Example 2 | | |
| | Average | n = 1 | n = 2 | Average | n = 1 | n = 2 |
| Al | 13.8 | 14.1 | 13.5 | 14.3 | 14.3 | 14.2 |
| K | 1.44 | 1.39 | 1.49 | 1.08 | 1.06 | 1.10 |
| P | 17.4 | 17.7 | 17.1 | 18.3 | 18.3 | 18.3 |
| S | 1.57 | 1.45 | 1.69 | 1.11 | 1.10 | 1.12 |
| Ca | 0.0041 | 0.0040 | 0.0042 | 0.0014 | 0.0014 | 0.0015 |
| Mg | 0.168 | 0.141 | 0.195 | 0.115 | 0.105 | 0.125 |
| Na | 3.33 | 3.08 | 3.58 | 3.32 | 3.12 | 3.51 |

[Table 3]

| Unit: mol | | | | |
|---|---|---|---|---|
| | Example 1 | | Example 2 | |
| | n = 1 | n = 2 | n = 1 | n = 2 |
| Al | 0.52 | 0.50 | 0.53 | 0.53 |
| P | 0.57 | 0.55 | 0.59 | 0.59 |

(continued)

| Unit: mol | | | | |
|---|---|---|---|---|
| | Example 1 | | Example 2 | |
| | n = 1 | n = 2 | n = 1 | n = 2 |
| Al/P | 0.91 | 0.91 | 0.90 | 0.89 |

(2) Measurement of cumulative pore volume, average pore diameter, bulk density, apparent density, and porosity

- Measurement method: Mercury porosimetry
- Pretreatment and measurement conditions:
  A sample was vacuum dried at room temperature for a whole day and night. Pore distribution of pore diameters of about 0.0036 to 200 μm was determined by mercury porosimetry. Pore size was calculated using the Washburn equation.

[Expression 1]

Washburn equation : $PD = -4\sigma\cos\theta$

P : Pressure  σ : Surface tension of mercury

D : Pore diameter  θ : Contact angle of mercury with sample

Surface tension of mercury : 480dynes/cm

Contact angle of mercury with sample : 140degrees

- Measuring device: Autopore IV 9520 (manufactured by micromeritics)
- Results: The measurement results are shown in Table 4 below.

[Table 4]

| | Example 1 | Example 2 |
|---|---|---|
| Cumulative pore volume (mL/g) | 1.02 | 1.35 |
| Average pore diameter (μm) | 0.036 | 0.050 |
| Bulk density (g/mL) | 0.68 | 0.55 |
| Apparent density (True density) (g/mL) | 2.18 | 2.07 |
| Porosity (%) | 69 | 74 |

(3) X-Ray diffraction

- Pretreatment and measurement conditions:

  A sample was crushed in an agate mortar, then filled into a sample cell (made of Al).

  X-Ray tube: CuKα

Optical system: Concentration method

Tube voltage/tube current: 45 kV-200 mA

Scan range: 5 to 80 deg

Scan step: 0.02 deg

Scan speed: 10 deg/min

Detector: One-dimensional semiconductor detector

- Measuring device: X-ray diffraction analyzer SmartLab (manufactured by Rigaku Corporation)
- Results: The measurement results are shown in Table 5 below. The measurement results were calculated from matching with a library pattern, assuming that the sample is composed of only the identified compounds.

[Table 5]

| | Compound content (%) | |
|---|---|---|
| | Example 1 | Example 2 |
| Amorphous | 91 | 95 |
| NaCl Halite (00-005-0628) | 9 | 5 |

(4) Thermogravimetric - mass spectrometry

- Pretreatment and measurement conditions:

  (TG-DTA)
  Pan: Pt (open type)
  Measuring range: RT to 1000°C
  Temperature rising rate: 10°C/min
  Atmosphere: $N_2$ 200 mL/min
  (MS)
  Ionization: EI
  Scanning range: m/z = 2 to 200

- Measuring device: Thermogravimetric-mass spectrometry simultaneous measurement device TG-DTA2020SA/MS9610 (manufactured by Bruker AXS)
- Results: The dehydration amount evaluation calculated from an ionic strength at m/z = 18 was 18% by weight in Example 1 and 17% by weight in Example 2.

(5) Electron microscope observation

- Measurement method: Field emission scanning electron microscopy (FE-SEM)
- Pretreatment and measurement conditions:
  A sample was taken, and platinum was vapor-deposited, then the surface of the sample was observed and photographed at 500 times and 5,000 times with FE-SEM.
- Measuring device: FE-SEM/EDX JSM-7500FA (manufactured by JEOL Ltd.)

The observation results are shown in Fig. 1. As shown in Fig. 1, fine irregularities were confirmed on the surfaces of the aluminum phosphate compounds of Example 1 and Example 2. It was confirmed that the particles present on the surface in some places were NaCl.

[Test Example 3: Purification of Protein with Histidine Tag]

His-Flag-fluorescent protein (mCherry: manufactured by Clontech Laboratories, Inc.) was expressed in Escherichia coli (E. coli BL21 (DE3): manufactured by Merck), and then suspended in 10 mM phosphate buffer (pH 7.3), followed by sonication. This gave a crude protein solution was obtained. Of this crude protein solution, a crude protein solution containing 10 mg as the amount of protein was suspended in 10 mM phosphate buffer (pH 7.3), and 300 mg of the aluminum phosphate compound prepared in Example 1 or 300 mg of commercially available aluminum phosphate (CAS:7784-30-7 Cat#: 11109 manufactured by Alfa Aesar) was mixed as a carrier to adsorb the protein.

[Table 6]

| | Protein | Carrier |
|---|---|---|
| Example 3 | Derived from E. coli | Aluminum phosphate compound of Example 1 |
| Comparative Example 1 | Derived from E. coli | Commercially available aluminum phosphate |

The carrier on which the protein was adsorbed was recovered, an eluate (a phosphate buffer solution added with 120 mM EDTA-3NA) was added, the protein adsorbed on the carrier was released into the eluate, and the eluate was collected. The obtained eluate is shown in Fig. 2. As shown in Fig. 2, it is recognized that Example 3 using the aluminum phosphate compound of the present invention as the carrier clearly shows strong fluorescence as compared with Comparative Example 1, and adsorbs a large amount of proteins as compared with Comparative Example 1.

The collected eluate was separated by SDS-polyacrylamide gel electrophoresis (SDS-PAGE). As a gel, a gel with an acrylamide concentration gradient (5 to 20% by weight gradient gel) was used, and the applied amount of the eluate was set to 17 μL. The results of electrophoresis are shown in Fig. 3. In Fig. 3, lane (i) shows a result of the crude protein solution before being adsorbed on the carrier, lane (ii) shows a result of Example 3, and lane (iii) shows a result of Comparative Example 1. A band indicated by an arrow corresponds to His-Flag-fluorescent protein. As can be seen from a comparison between the lane (ii) (Example 3) and the lane (iii) (Comparative Example 1) in Fig. 3, it was shown that commercially available aluminum phosphate did not sufficiently function as the carrier for protein purification, whereas the aluminum phosphate compound of the present invention is suitable as the carrier for protein purification.

[Test Example 4: Purification of Protein without Histidine Tag]

A pneumococcal surface protein was expressed in Escherichia coli (E. coli BL21 (DE3): manufactured by Merck), then suspended in 10 mM phosphate buffer (pH 7.3), and crushed using a pressure homogenizer. This gave a crude protein solution was obtained. Of this crude protein solution, a crude protein solution containing 4 mg as the amount of protein was suspended in 30 mM phosphate buffer (pH 6.75) containing a surfactant and sodium chloride, and 120 mg of the aluminum phosphate compounds prepared in Examples 1 and 2 were mixed as carriers to adsorb the protein.

[Table 7]

| | Protein | Carrier |
|---|---|---|
| Example 4 | Derived from pneumococcus | Aluminum phosphate compound of Example 1 |
| Comparative Example 5 | Derived from pneumococcus | Aluminum phosphate compound of Example 2 |

The carrier on which the protein was adsorbed was recovered, an eluate (a phosphate buffer solution added with 120 mM EDTA-3NA) was added, the protein adsorbed on the carrier was released into the eluate, and the eluate was collected. Further, the collected eluate was separated by SDS-polyacrylamide gel electrophoresis (SDS-PAGE). As a gel, a gel with a uniform concentration of acrylamide (12.5% gel) was used, and the applied amount of the eluate was set to 17 μL. The results of electrophoresis are shown in Fig. 4. In the figure, lane (i) shows a result of Example 4 (using the aluminum phosphate compound of Example 1 as the carrier), and lane (ii) shows a result of Example 5 (using the aluminum phosphate compound of Example 2 as the carrier). A band indicated by an arrow corresponds to the pneumococcal surface protein. As can be seen from a comparison between the lane (i) and the lane 2 (ii) in Fig. 4, it was shown that the protein purification efficiency was better when the aluminum phosphate compound of Example 1 was used as the carrier.

Although endotoxin derived from Escherichia coli poses a serious problem for quality control of pharmaceuticals, endotoxin derived from Escherichia coli was not detected in the collected eluate in this test example. That is, it was found that the aluminum phosphate compound of the present invention also can effectively remove the endotoxin derived from Escherichia coli.

## Claims

1. A method for producing an aluminum phosphate compound, comprising a step for mixing an aqueous solution A that contains a phosphate ion and an aqueous solution B that contains an aluminum ion and a sulfate ion and at least one of a potassium ion or a magnesium ion, to obtain a mixed solution containing an aluminum phosphate compound.

2. The method for producing an aluminum phosphate compound according to claim 1, wherein the mixed solution has a pH of 3 to 4.

3. The method for producing an aluminum phosphate compound according to claim 1 or 2, wherein the aqueous solution A is an aqueous monohydrogen phosphate solution.

4. The method for producing an aluminum phosphate compound according to any one of claims 1 to 3, wherein the aqueous solution B contains the potassium ion and the magnesium ion.

5. The method for producing an aluminum phosphate compound according to any one of claims 1 to 4, wherein the aqueous solution B is prepared by dissolving at least one of potassium aluminum sulfate or magnesium sulfate in water.

6. The method for producing an aluminum phosphate compound according to any one of claims 1 to 5, wherein the mixing is performed by adding the aqueous solution B to the aqueous solution A at a rate of 2% by volume/min or less.

7. The method for producing an aluminum phosphate compound according to any one of claims 1 to 6, wherein the aluminum phosphate compound is used for a carrier for protein purification.

8. An aluminum phosphate compound obtained by the method for producing an aluminum phosphate compound according to any one of claims 1 to 7.

9. An aluminum phosphate compound comprising sulfur and at least one of 0.5% by weight or more of potassium or 0.01% by weight or more of magnesium, together with aluminum phosphate.

10. The aluminum phosphate compound according to claim 9, wherein a content of sulfur is 0.5% by weight or more.

11. The aluminum phosphate compound according to claim 9 or 10, wherein a content of aluminum is 10 to 16% by weight.

12. The aluminum phosphate compound according to any one of claims 9 to 11, wherein a water content is 15% by weight or more.

13. The aluminum phosphate compound according to any one of claims 9 to 12, wherein a content of calcium is 0.001% by weight or more.

14. The aluminum phosphate compound according to any one of claims 9 to 13, which is used as a carrier for protein purification.

15. The aluminum phosphate compound according to any one of claims 9 to 14, which is used for purification of a protein synthesized by a cell-free system, and/or a protein expressed in a host selected from the group consisting of bacteria, yeast, algae, an insect cell, a mammalian cell, and an animal cultured cell.

16. The aluminum phosphate compound according to any one of claims 9 to 15, which is used for purification of a protein expressed in Escherichia coli as a host.

FIG. 1

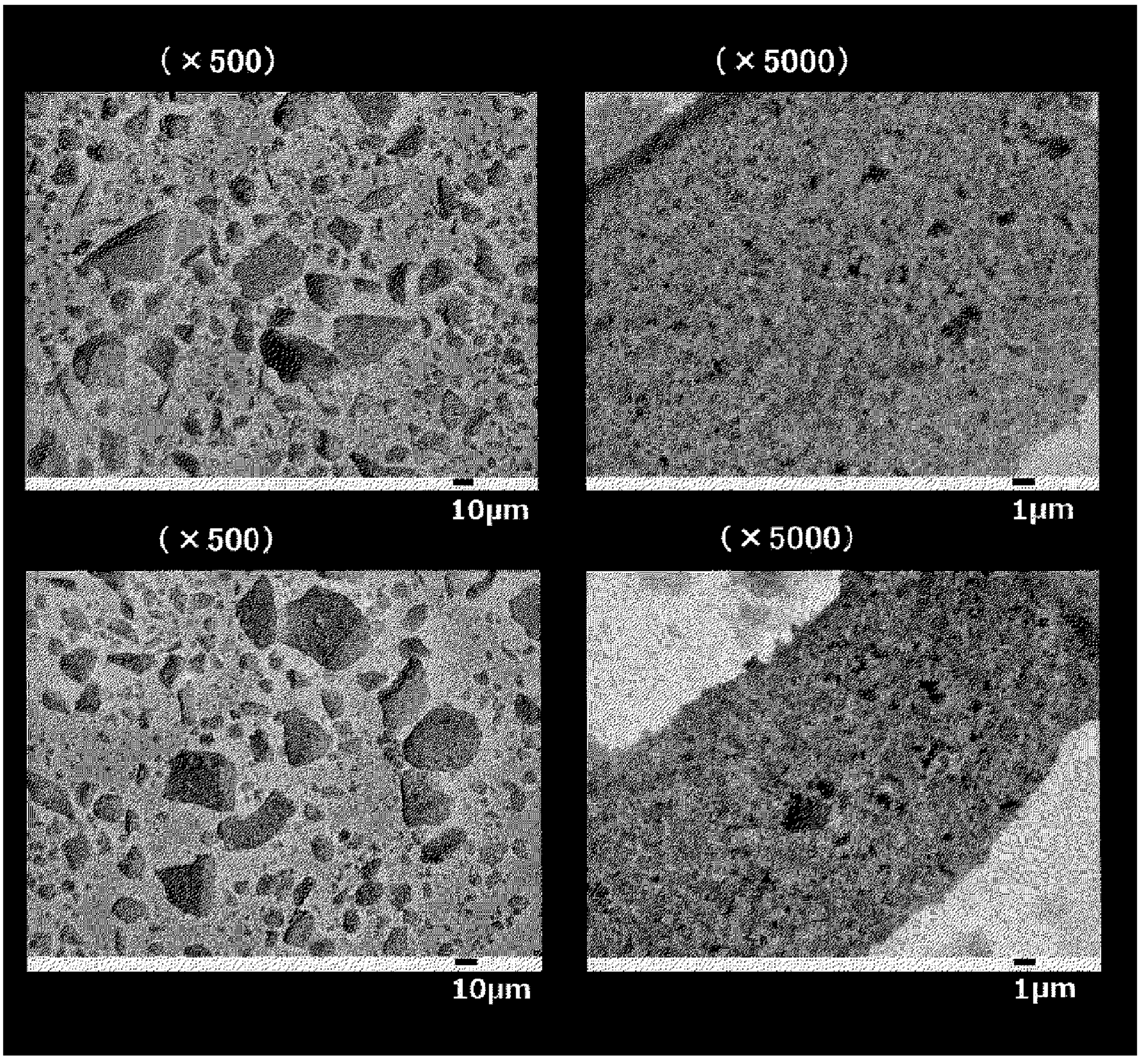

FIG. 2

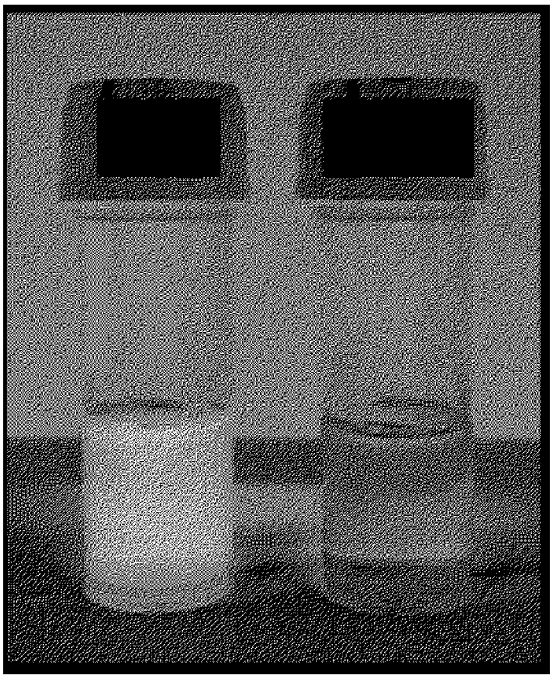

FIG. 3

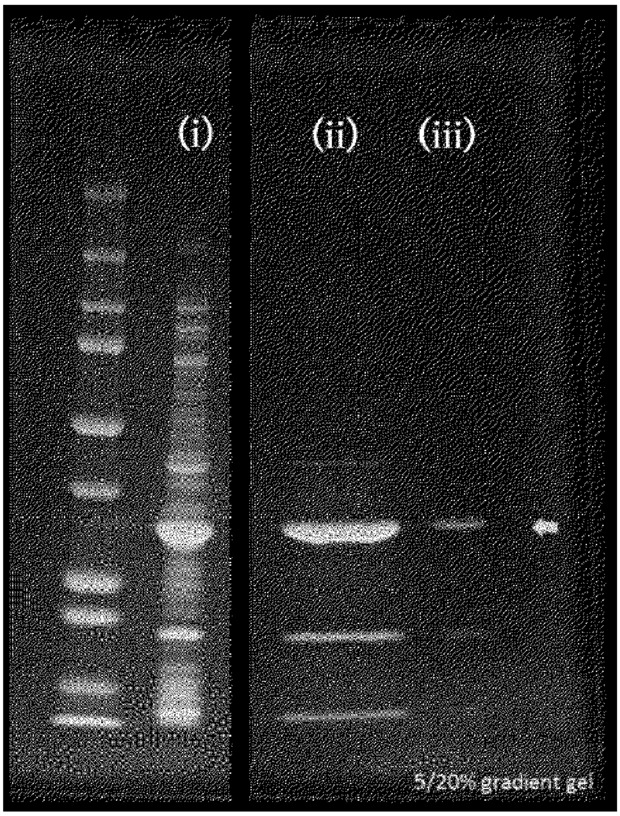

FIG. 4

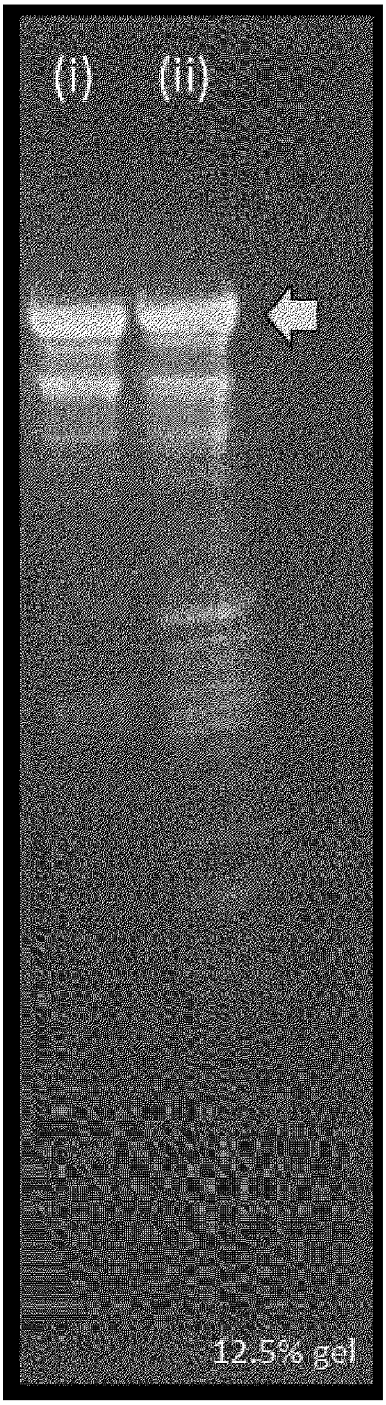

## INTERNATIONAL SEARCH REPORT

International application No.
PCT/JP2019/020340

A. CLASSIFICATION OF SUBJECT MATTER
Int.Cl. C01B25/36(2006.01)i, B01J20/02(2006.01)i, B01J20/28(2006.01)i, B01J20/30(2006.01)i

According to International Patent Classification (IPC) or to both national classification and IPC

B. FIELDS SEARCHED

Minimum documentation searched (classification system followed by classification symbols)
Int.Cl. C01B25/36, B01J20/02, B01J20/28, B01J20/30

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN), JSTPlus (JDreamIII), JST7580 (JDreamIII), JSTChina (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | KLEIN, J., et al., "Analysis of Aluminum Hydroxyphosphate Vaccine Adjuvants by $^{27}$Al MAS NMR", Journal of Pharmaceutical Sciences, March 2000, vol. 89, no. 3, pp. 311-321, in particular, page 313, Batch Precipitations | 1, 5, 8-13<br>3<br>2, 4, 6, 7, 14-16 |
| X<br>Y<br>A | CN 102249207 A (WANG, Li) 23 November 2011, example 1 (Family: none) | 1, 5, 8-13<br>3<br>2, 4, 6, 7, 14-16 |
| Y | US 2014/0314653 A1 (THIRIOT, David S.) 23 October 2014, paragraphs [0084], [0100] & WO 2013/078102 A1 & KR 10-2014-0100530 A | 3 |
| Y | JP 2011-020892 A (RYOKOLIME INDUSTRY CO., LTD.) 03 February 2011, paragraph [0085] (Family: none) | 3 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 July 2019 (16.07.2019) | 30 July 2019 (30.07.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)